(19) Europäisches Patentamt
European Patent Office
Office européen des brevets

(11) EP 1 886 658 A2

(12) # EUROPEAN PATENT APPLICATION

(43) Date of publication:
**13.02.2008 Bulletin 2008/07**

(51) Int Cl.:
***A61K 6/00*** *(2006.01)*

(21) Application number: **07015296.2**

(22) Date of filing: **03.08.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **08.08.2006 IT MI20061595**

(71) Applicant: **Giovanni Ogna & Figli S.p.A.**
**20053 Taccona di Muggio MI (IT)**

(72) Inventor: **Taborelli, Corrado**
**20038 Seregno (MI) (IT)**

(74) Representative: **Pipparelli, Claudio et al**
**PIPPARELLI & PARTNERS**
**Via Quadronno, 6**
**20122 Milano (IT)**

(54) **Gel for canal irrigation based on sodium hypochlorite**

(57) The use is described of an aqueous formulation in the form of a gel, as a lubricant and disinfectant for canal irrigation, comprising:
a) sodium hypochlorite;
b) water;
the above aqueous formulation, in the form of a gel, being characterized in that it includes a viscosifying composition selected from:
1) amine oxides having general formula

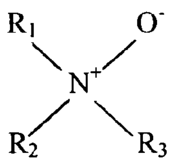

wherein $R_1$ and $R_2$, the same or different, are selected from methyl and ethyl; $R_3$ is an alkyl radical from $C_{10}$ to $C_{18}$, preferably from $C_{12}$ to $C_{14}$;
in combination with salts of alkaline or alkaline earth metals, preferably alkaline, of saturated fatty acids;
2) ethylene oxide-propylene oxide block copolymers;
3) polymers based on polyacrylic acid;
said aqueous formulation in the form of a gel being further characterized by a viscosity at 25°C of 800 to 6,000 cP, preferably from 1,000 to 2,500 cP.
The presentation is also described in glass (or other inert material) syringes of the aqueous formulation in the form of a gel of the present invention, the above syringes being used by odontological doctors in canal treatment. Said syringes can be equipped separately with a washing needle made of steel or plastic material, with a blunted tip.

EP 1 886 658 A2

**Description**

FIELD OF THE INVENTION

The present invention belongs to the field of medical devices (according to dir. 93/42/CE) for the exclusive use of odontological doctors for endo-canal treatment.

PRIOR ART

When the inner space of a tooth, containing the vital dental pulp, loses its insulation from the surrounding oral environment, it can be infiltrated by micro-organisms whose proliferation causes inflammation and progressive pulp degeneration with the relevant appearance of pain and other symptomatologies. It is therefore necessary to carry out an endodontic intervention aimed at preserving the dental element which can still be recovered.

The main objective of endodontic treatment is the cleaning of the pulp chamber and of the infected endodontic radicular canals, their disinfection, shaping and subsequent sealing. The internal anatomy of teeth is complex and consists, in addition to a main canal for each root, of a series of canals and side openings.

The cleaning phase of the radicular canal system is consequently rather delicate due to the complex anatomy of the endodontic cavities, and requires the use of both mechanical instrumentation which mechanically removes the tissues, and irrigation liquids which facilitate the instrumentation action and are capable of penetrating all the branches of the radicular canal system, even in points which cannot be reached by mechanical instrumentation.

The purpose of the irrigation can therefore be summarized in the following points:

- Reduction in the bacterial charge present inside the radicular canal system;
- Dissolution of the necrotic tissues present;
- Lubrication of the canal instruments;
- Evacuation of the fragments caused by the mechanical instrumentation (smear layer)

It is currently impossible to achieve all the irrigation objectives in an optimal way using only one irrigator: present practice includes the alternating use of various solutions dif fering in their chemical nature and consequently in their activity, whose use globally achieves the objectives of the treatment.

Among the various chemical solutions currently used, sodium hypochlorite at different concentrations is certainly the most common and appreciated as a result of its interesting properties.

Sodium hypochlorite (CAS 8007-59-8) is a chemical substance which can be defined as sodium salt of the hypochlorous acid present in numerous chemical compounds of common use, among which bleach.

In the odontological field, it has been used since the forties' as an irrigating solution, by Walker, who demonstrated its characteristic as a solvent of organic substances and an antiseptic within a wide range.

Over the years, numerous studies have confirmed its disinfectant properties: sodium hypochlorite can kill vegetative bacteria, spores, fungi, protozoan and viruses (including HIV, the viruses of hepatitis A and B).

In addition to a strong antimicrobial activity, sodium hypochlorite has a dissolvent activity towards organic and in particular necrotic tissues, present in infected radicular canals. This characteristic activity is specific of hypochlorite which other solutions used for canal irrigation do not have.

Both the antibacterial and digestive activity of organic tissues can be incremented by a possible heating before application.

Hypochlorite solutions have an optimum dissolution capacity of organic tissues but a poor suspensive activity of the inorganic fragments and therefore a more difficult subsequent removal. The necessity is therefore felt for using sodium hypochlorite in the form of a gel, with a consequent improvement in the suspensive capacity of the inorganic fragments.

From a technological point of view, it is not simple to prepare a gel based on sodium hypochlorite which is stable and adequate for use in the endodontic field: it is known, in fact, that the oxidative power and alkalinity of sodium hypochlorite make this chemical compound incompatible with most of the substances commonly used for gelifying solutions.

US2003/0156980 attempted to overcome this problem by preparing a viscous disinfecting composition based on aqueous sodium hypochlorite and a thickening agent, in particular "fumed silica" which is a particular anhydrous silica. The above-mentioned composition, initially having a low viscosity, increases its viscosity with time until a gel is formed. In this way, the disinfectant composition can adhere better to the walls of the tooth treated.

The solution proposed by US 2003/156980, however, has the disadvantage that "fumed silica" in aqueous dispersion has a very high pH, between 3 and 5. Consequently, high quantities of a pH regulator must be used in order to obtain a strongly basic pH, necessary for stabilizing the sodium hypochlorite solution. As a consequence, there is a

decrease in the rheological stability of the composition and in the stability of the chlorine titre.

A gel based on sodium hypochlorite has now been found, and this represents the object of the present invention, which satisfies the above requirements, in particular the stability with respect to time.

According to the above, a first object of the present invention relates to the use as lubricant and disinfectant for canal irrigation of an aqueous formulation in the form of a gel, comprising:

a) sodium hypochlorite;
b) water;
the above aqueous formulation, in the form of a gel, being characterized in that it includes a viscosifying composition selected from:

1) amine oxides having general formula

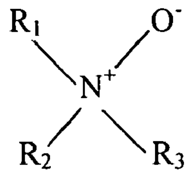

wherein R, and $R_2$, the same or different, are selected from methyl and ethyl, preferably methyl; $R_3$ is an alkyl radical from $C_{10}$ to $C_{18}$, preferably from $C_{12}$ to $C_{14}$; in combination with salts of alkaline or alkaline earth metals, preferably alkaline, of saturated fatty acids;
2) ethylene oxide-propylene oxide block copolymers;
3) polymers based on acrylic acid;

said aqueous formulation in the form of a gel being further characterized by a viscosity at 25°C ranging from 800 to 6,000 cP, preferably from 1,000 to 2,500 cP.

In the aqueous formulation in the form of a gel of the present invention, sodium hypochlorite is present in a quantity ranging from 0.5% to 10% as available chlorine, preferably from 2 to 8%; even more preferably from 4 to 6%. The viscosifying composition is present in a quantity ranging from 0.2 to 20% by weight, preferably from 2 to 14%, even more preferably from 5 to 10% by weight; the complement to 100 being water.

As far as the polymers based on acrylic acid are concerned, these are crosslinked polymers of acrylic acid, with polyvinyl ethers or diacrylic acids. The above polymers are normally stabilized with aromatic compounds, for example those described in US-A-5,997,764 and US-A-6,083,422.

With respect to ethylene oxide/propylene oxide block copolymers (CAS 9003-11-6 or 106392-12-5), these are well known to experts in the field. They are non-ionic surface-active agents (or surfactants), stable in both an acid and basic environment. Particularly used in the cosmetic and pharmaceutical field, they thicken the aqueous solutions forming gels with a varying density in relation to their molecular weight and concentration.

In the preferred embodiment, the viscosifier is represented by composition 1. In this case the amine oxide, the aqueous formulation in the form of a gel being 100, is present in a quantity ranging from 0.1 to 10% by weight, preferably from 1 to 7% by weight, even more preferably from 3 to 6% by weight. The salts of fatty acids, on the other hand, are contained in a quantity ranging from 0.1 to 10% by weight, preferably from 1 to 7% by weight, even more preferably from 2 to 4% by weight.

As far as the amine oxides are concerned, these are so-called amphoteric surfactants. Typical but non-limiting examples of these amphoteric surfactants are N,N-dimethyl dodecyl amine N-oxide, N,N- dimethyl tetradecyl amine N-oxide, N,N-dimethyl stearyl amine N-oxide, N,N-dimethyl decyl amine N-oxide, N,N-dimethyl cetyl amine N-oxide. Amine oxides wherein $R_3$ is a mixture of $C_{10}$ and $C_{20}$ alkyls, preferably from $C_{12}$ to $C_{16}$ can obviously be used in the present invention. In the preferred embodiment the amine oxide is N,N-dimethyl dodecyl amine N-oxide (CAS[1643-20-5]).

The saturated fatty acids have the general formula $C_nH_{2n}O_2$ with n ranging from 8 to 30, preferably from 14 to 20. The salts of fatty acids are also surfactants, but of the anionic type. Typical examples of the above saturated acids are octanoic acid (or caprylic acid) with n=8, decanoic acid (or capric acid) with n=10, dodecanoic acid (or lauric acid) with n=12, tetradecanoic acid (or myristic acid) with n=14, hexadecanoic acid (or palmitic acid) with n=16, octadecanoic acid (or stearic acid) with n=18, eicosanoic acid (or arachidic acid) with n=20.

In the preferred embodiment, the saturated fatty acids are in the form of salts of alkaline metals, preferably selected from Na and K. The saponification product of coconut oil with soda or potash can be efficaciously used. The

above salt will have the general formula $RCOO^-M^+$ wherein R is a $C_{14}$-$C_{20}$ alkyl radical and $M^+$ is selected from $K^+$ and $Na^+$.

As far as the water is concerned, this is water purified by distillation in order to eliminate all possible substances which can alter the stability of the hypochlorite.

The term gel means a colloidal composition having a gelatinous aspect. The disinfecting and lubricating gel of the present invention has a viscosity which depends on its composition. The higher its water content, obviously the lower its viscosity will be. The above viscosity was determined at 25°C by means of a Brookfield RV revolving 2.20 rpm viscosimeter. The term disinfectant generally means a substance (or formulation) capable of killing, complexing or destroying bacteria in some way.

The formulations of the present invention are stable with time and have a very low titre loss during the shelf-life period of the product (24 months) if kept in the refrigerator (about 4 °C) and protected from light. The experimental part shows the stability of the compositions of the present invention. The above stability is determined by measuring the variation in the active chlorine of the gel of the present invention.

In this respect, it should be noted that sodium hypochlorite is commercially available only in aqueous solutions having various titres; it is in fact synthesized by means of the reaction:

$$Cl_2 + 2\ NaOH \rightarrow NaOCl + NaCl + H_2O + heat$$

There are several ways for expressing the sodium hypochlorite concentration: the most widely used is "available chlorine".

If we examine the following reactions:

$$NaOCl + 2\ KI + 2\ CH_3COOH \rightarrow I_2 + NaCl + 2\ CH_3COOK + H_2O$$

$$Cl_2 + 2\ KI \rightarrow I_2 + 2\ KCl$$

it can be seen that a molecule of sodium hypochlorite has the same oxidative capacity as a chlorine molecule, as both oxidize two molecules of potassium iodide.

The expression which connects the weight percentage of hypochlorite with the "available chlorine" is given by the following relationship:

$$\text{wt \% NaOCl} = (\text{\% av.Cl}) \cdot \frac{\text{mol. wt NaOCl}}{\text{mol. wt } Cl_2} = (\text{\% av. Cl}) \cdot \frac{74.44}{70.91} = 1.05$$

therefore the weight % of NaOCl is obtained from the available chlorine % multiplied by 1.05.

Returning to the compositions of the present invention, as a result of their viscosities, they minimize the possibility of accidental leakages. These are much more probable in the presence of aqueous solutions (less viscous) of sodium hypochlorite, with the risk of contact of the hypochlorite with the patient or the dentist in particularly sensitive areas of the body, such as the eyes, with consequent irritation or even damage.

Sodium hypochlorite in solution also has a reasonable lubricating activity for the instruments, which can be further enhanced if used together with surfactants.

The introduction of surfactants in the preferred formulations of the present invention also has two more advantages: it decreases the surface tension of the product, favouring its penetration in points which cannot be reached by the instruments and it gives the product a suspension capacity for the fragments formed during the instrumentation.

This latter aspect is particularly interesting for odontological techniques: whereas the dissolution capacity of organic tissues is known and proved, it is also known that hypochlorite has a poor dissolution activity of the inorganic tissue. Only the use of non-liquid but gel irrigation solutions, as in the present invention, provide an improvement in the suspensive capacity of the inorganic fragments and therefore an easier subsequent removal of the same.

**Product presentation**

As far as the application technique of sodium hypochlorite is concerned, all the preparations currently on the market present sodium hypochlorite in bottles, with an active chlorine titre ranging from 0.5 to 5.25%. The highest concentrations, over 2%, are corrosive for the tissues and this clinical datum is important considering the operative sequence the doctor has to adopt:

- preparation of the operating table with dike and sucker
- opening of the flask
- preparation of a suitable syringe with needle
- insertion of the needle into the flask
- suction of the liquid
- treatment of the patient with the liquid.

This working sequence is particularly delicate as accidental spills of solution are possible, which can accidentally come into contact with the patient or dentist in particularly sensitive areas of the body, such as the eyes, with consequent irritation or even damage.

The moment in which the liquid is applied to the patient is of critical importance, but it is fundamental for a satisfactory result of the operation to have previously prepared the syringe and needle suitable for the canal irrigation. Also the manipulation of bottles, of ten made of glass, can involve the risk of spills or accidental falls, which can cause accidents.

A presentation of sodium hypochlorite in a syringe, with the relative needle which must be assembled by the dentist before using the product on the patient, is therefore of great interest. This presentation is even more important if the product is in the form of a gel, which is easier to weigh and less mobile than a liquid.

The presentation of a syringe and needle in a kit can prevent possible accidents. It is in fact known that it is not necessary to exert an excessive force, or try to force the needle when a canal irrigation is effected with sodium hypochlorite. Even with all possible care, however, if a needle for injections is unintentionally used, it is highly probable that a radicular perforation or an apical leakage will occur.

The formulation of the present invention is parcelled in syringes, ready for use by the doctor, who simply has to insert a needle, preferably supplied in the package, with a blunted tip.

A further object of the present invention therefore relates to syringes for canal irrigation, made of inert material, preferably glass, containing the composition according to claim

1. The syringes are separately equipped with a washing needle made of steel (or plastic material) with a blunted tip.

The following examples are provided for a better understanding of the present invention.

## EXAMPLES

In examples 1 and 2 the amine oxide is N,N-dimethyl dodecyl amine N-oxide, whereas the potassium salts of fatty acids consist of a mixture of potassium alkyl carboxylates, obtained by the saponification of coconut oil.

### Example 1

| | |
|---|---|
| Sodium hypochlorite | 5% as available chlorine |
| Amine oxide | 4.5% |
| Potassium salts of fatty acids | 3% |
| Water | complement to 100 |

The preparation was obtained as follows:

13.5 g of amine oxide (Laucosol ox, Industrie Chimiche Panzeri) and 9.0 g of potassium salts of fatty acids (Neopon K, Industrie Chimiche Panzeri), were weighed in a 400 ml beaker. The pre-established quantity of water is added (50.7 g) and the whole mixture is stirred until a limpid and homogeneous gel is obtained. Finally, sodium hypochlorite is added (226.8 g of a solution having an active chlorine titre of 6.62%). The whole mixture is hand-stirred until completely homogenized.

A limpid, straw-coloured gel is obtained, with the characteristic odour of chlorine. pH > 12.5; viscosity at 25°C 1,300 cP.

The product was divided into I type glass syringes having a capacity of 3 ml, with a Tef lon closing.

The gel obtained has a titre as active chlorine of 5.12%. The titre as active chlorine is stable for 1 year from the preparation if kept in a refrigerator at +4°C (The titre passes from 5.12 to 4.3).

### Example 2

| | |
|---|---|
| Sodium hypochlorite | 4.5% as active chlorine |
| Amine oxide | 4.0% |
| Potassium salts of fatty acids | 3% |
| Water | complement to 100 |

This example was prepared with the same procedure as the previous example. The gel thus obtained (viscosity at 25°C of 1,100 cP) has a titre of active chlorine which, after 12 months from its preparation and kept in a refrigerator at 4°C, shows a reduction in the active chlorine titre of only 10%.

### Example 3

Poloxamer 407 (CAS Nr. 9003-11-6) commercialized by BASF under the trade-mark Pluronic® F 127, was used as ethylene oxide/propylene oxide block copolymer.

| | |
|---|---|
| Sodium hypochlorite | 4% as active chlorine |
| Poloxamer 407 | 20% |
| Water | complement to 100g |

The preparation of the product is effected in two phases: a gel of Poloxamer 407 50% w/w in water is prepared on the one hand, using a turbo-emulsifier which dissolves the Poloxamer, under vacuum. The necessary quantity of water is charged first and Poloxamer (as flakes) is subsequently sucked in. After about one hour of mixing a high viscosity gel is obtained (> 20,000 cP at 25°C) which is subsequently diluted in the machine with the quantity of hypochlorite necessary for obtaining the desired titre in chlorine.

The final product is a limpid straw-coloured gel, having a viscosity of about 5,000 cP at 25°C.

This formulation proves to be stable when kept at +4°C, for at least one year.

### Example 4

| | |
|---|---|
| Sodium hypochlorite | 4.0% as active chlorine |
| Sodium salt of Carbomero (Carbopol® | 676) 2.0% |
| Oxy-rite® 100 | 0.30% |
| Water | complement to 100g |

The preparation of the product is as follows: the necessary quantity of water is charged under vacuum into a turbo-emulsifier, Carbopol is dispersed until a viscous solution is obtained and soda is then added. Oxy-rite® 100 is subsequently added (stabilizer of Carbopol) and finally sodium hypochlorite.

A fluid gel, having a viscosity of about 1,500-2,000 cP at 25°C, pH > 12.5, is obtained.

The stability of this preparation is similar to that found in the other preparations: after 1 year, kept at +4°C, the titre decreased by only 15%.

## Claims

**1.** Use as lubricant and disinfectant for canal irrigation of an aqueous formulation in the form of a gel, comprising:

a) sodium hypochlorite;
b) water;
the above aqueous formulation, in the form of a gel, being **characterized in that** it includes a viscosifying composition selected from:

1) amine oxides having general formula

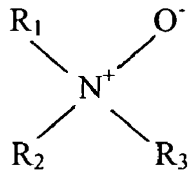

wherein $R_1$ and $R_2$, the same or different, are selected from methyl and ethyl, preferably methyl; $R_3$ is an alkyl radical from $C_{10}$ to $C_{18}$, preferably from $C_{12}$ to $C_{14}$; in combination with salts of alkaline or alkaline earth metals, preferably alkaline, of saturated fatty acids;
2) ethylene oxide-propylene oxide block copolymers;
3) polymers based on acrylic acid;

said aqueous formulation in the form of a gel being further **characterized by** a viscosity at 25°C ranging from 800 to 6,000 cP, preferably from 1,000 to 2,500 cP.

**2.** The use according to claim 1, wherein sodium hypochlorite is present in quantities ranging from 0.5% to 10% as available chlorine, preferably from 2 to 8%, even more preferably from 4 to 6%;
the viscosifying composition is present in a quantity ranging from 0.2 to 20% by weight, preferably from 2 to 14% by weight, even more preferably from 5 to 10% by weight;
the complement to 100 being water.

**3.** The use according to claim 1, wherein the viscosifying composition is selected from amine oxides, in combination with salts of alkaline metals of fatty acids.

**4.** The use according to claim 3, wherein, the aqueous formulation in the form of a gel being 100;
the amine oxide is present in quantities ranging from 0.1 to 10% by weight, preferably from 1 to 7% by weight, even more preferably from 3 to 6% by weight;
the salts of the saturated fatty acids are present in quantities ranging from 0.1 to 10% by weight, preferably from 1 to 7% by weight, even more preferably from 2 to 4% by weight.

**5.** The use according to claim 3, wherein the viscosifying composition comprises:

N,N-dimethyl dodecyl amine N-oxide;
the saponification product of coconut oil with potash.

**6.** Syringes for canal irrigation in inert material, preferably glass, containing the composition according to claim 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030156980 A
- US 2003156980 A
- US 5997764 A
- US 6083422 A